# EUROPÄISCHE PATENTSCHRIFT

(11) **EP 1 400 493 B1**
(45) Veröffentlichungstag und Bekanntmachung des Hinweises auf die Patenterteilung: **29.12.2010**
(21) Anmeldenummer: 03019547.3
(22) Anmeldetag: 29.08.2003
(51) Int. Cl.: C02F 1/30, C02F 1/72, C02F 1/74, A61L 2/08, B01J 19/08, A62D 3/00

(54) **Verfahren zur Reinigung einer schadstoffbelasteten Flüssigkeit**
Process for cleaning a contaminated liquid
Procédé pour la purification d'un liquide contaminé

(30) Priorität: 17.09.2002 DE 10243799
(43) Veröffentlichungstag der Anmeldung: 24.03.2004
(73) Patentinhaber: Deutsches Zentrum für Luft- und Raumfahrt e.V., 51147 Köln (DE)
(72) Erfinder: Renz, Günther, Dr., 70794 Filderstadt (DE); Nedele, Martin, Dr., 72768 Reutlingen (DE)
(74) Vertreter: Hoeger, Stellrecht & Partner Patentanwälte

(56) Entgegenhaltungen:
- EP-A- 0 931 765
- WO-A-02/02466
- DE-C- 19 740 401
- PATENT ABSTRACTS OF JAPAN vol. 014, no. 427 (C-0758), 13. September 1990 (1990-09-13) & JP 02 164490 A (JAPAN ATOM ENERGY RES INST;OTHERS: 02), 25. Juni 1990 (1990-06-25)
- PATENT ABSTRACTS OF JAPAN vol. 1998, no. 11, 30. September 1998 (1998-09-30) & JP 10 165970 A (HITACHI LTD), 23. Juni 1998 (1998-06-23)
- PIKAEV A K ET AL: "Electron-beam treatment of highly-coloured river water" RADIATION PHYSICS AND CHEMISTRY, ELSEVIER SCIENCE PUBLISHERS BV., AMSTERDAM, NL, Bd. 48, Nr. 1, 1. Juli 1996 (1996-07-01), Seiten 75-80, XP004050953 ISSN: 0969-806X

## Beschreibung

Die Erfindung betrifft ein Verfahren zur Reinigung einer schadstoffbelasteten Flüssigkeit, bei dem die Flüssigkeit mit Elektronen bestrahlt wird und vor der Elektronenbestrahlung Gas in die Flüssigkeit eingeblasen wird.

Eine Vorrichtung zur Reinigung einer schadstoffbelasteten Flüssigkeit, mit einer Elektronenquelle zur Beaufschlagung der Flüssigkeit mit Elektronen ist beispielsweise aus der DE 199 01 058 A1 bekannt.

Es tritt oftmals das Problem auf, dass Flüssigkeiten und insbesondere Wasser schwer abbaubare Schadstoffe und Schadstoffgemische wie Fluor-ChlorKohlenwasserstoffe und weitere organische und metall-organische Verbindungen enthält. Dies ist beispielsweise der Fall bei Sickerwasser aus Sondermülldeponien und Hausmülldeponien. Die Flüssigkeit kann auch biologisch aktive Substanzen enthalten und muss sterilisiert werden.

Aus der WO 02/02466 A1 ist ein Verfahren zur Aufbereitung von mit Schadstoffen belasteten pumpfähigen Medien bekannt, bei dem das aufzubereitende Medium in einen Vorreaktionsbehälter eingeleitet wird, dem ozonhaltiges Gas zugeführt wird, das Medium von dem Vorreaktionsbehälter in einen Hauptreaktionsbehälter übergeleitet wird, dem ozonhaltiges Gas zugeführt wird, und das Medium in dem Hauptreaktionsbehälter mit Elektronen bestahlt wird. Das Medium wird dann von dem Hauptreaktionsbehälter in einen von dem Vorreaktionsbehälter separaten Nachreaktionsbehälter übergeleitet, welchem ozonhaltiges Gas zugeführt wird.

Aus der EP 0 931 765 A2 ist ein Verfahren zur Konversion von mit gentoxischen Substanzen belastetem Wasser bekannt, bei dem der Wasserstrom direkt oder nach dem Durchlaufen einer Vorbehandlungs-Stufe mit Ozon angereichert wird und der Wasserstrom einer Einwirkung schneller Elektronen unterworfen wird.

Aus der JP 02164490 ist ein Wasserbehandlungsverfahren bekannt, bei dem Ozon oder sauerstoffhaltiges Gas in Wasser gelöst wird und das Wasser mit einem Elektronenstrahl beaufschlagt wird.

Aus der JP 10165970 ist eine Einrichtung zur Behandlung von Wasser mit Ozon bekannt.

Der Artikel "ELECTRON-BEAM TREATMENT OF HIGHLY-COLOURED RIVER WATER" von A.K. Pikaev et al., Radiat, Phys. Chem. Vol. 48, No. 1, Seiten 75 - 80, 1996 beschreibt eine Studie, in der die Reinigung von Wasser mit Elektronenbeaufschlagung studiert wurde.

Davon ausgehend liegt der Erfindung die Aufgabe zugrunde, ein Verfahren der eingangs genannten Art bereitzustellen, mit dem sich eine hocheffiziente Reinigung erzielen lässt.

Die eingangs genannte Aufgabe wird bei dem gattungsgemäßen Verfahren erfindungsgemäß dadurch gelöst, dass die Flüssigkeit als Film einer Filmdicke im Millimeterbereich geführt wird, dass die Flüssigkeit vor der Gaseinblasung verwirbelt wird, dass das Gas zur Ausbildung einer Zweiphasenströmung eingeblasen wird und dass die Zweiphasenströmung mit den Elektronen bestrahlt wird.

Insbesondere ist es vorteilhaft, wenn ein Gasvolumenstrom eingeblasen wird, der mindestens 10 % des Flüssigkeitsvolumenstroms beträgt. Dadurch erhält man eine effektive Zweiphasenströmung mit einem genügend großen Anteil an Gasblasen, um die Wechselwirkung mit dem Elektronenstrahl zu erhöhen und damit die Abbaurate zu erhöhen. Weiterhin wird, wenn Luft und/oder Sauerstoff eingeblasen wird, eine genügende Anzahl an Gasblasen bereitgestellt, um so eine genügend hohe Rate für die Erzeugung von aktiviertem Sauerstoff zu erhalten.

Günstig ist es, wenn bezogen auf die Strömungsrichtung der Flüssigkeit der Elektronenquelle eine Gaseinbringvorrichtung vorgeschaltet ist, mittels der in die Flüssigkeit Gas zur Ausbildung einer Zweiphasenströmung einblasbar ist.

Durch Einblasen eines Gases über die Gaseinbringvorrichtung lässt sich in der Flüssigkeit, die als Flüssigkeitsfilm geführt ist, eine Zweiphasenströmung ausbilden. Weiterhin wird durch das Einblasen von Gas die Flüssigkeit verwirbelt. Dadurch kann der Elektronenstrahl besser Schadstoffe in der Flüssigkeit abbauen, da effektiv die Eindringtiefe des Elektronenstrahls in die Flüssigkeit erhöht wird und damit eben dessen Abbaurate zunimmt.

Insbesondere lässt sich über die Gaseinbringvorrichtung ein reaktives Medium einblasen. Es kann sich dabei um Luft und/oder Sauerstoff handeln. Durch das reaktive Medium können Schadstoffe durch chemische Reaktionen abgebaut werden. Zusätzlich zu dem direkten Abbau von Schadstoffmolekülen kann dann der Elektronenstrahl an oder in Luftblasen bzw. an oder in Sauerstoffblasen, wenn Luft oder Sauerstoff eingeblasen wird, hochreaktives Ozon und/oder hochreaktive Sauerstoffradikale lokal erzeugen. Diese wiederum greifen Schadstoffmoleküle an und führen zu deren Abbau. Durch die Elektronenstrahlbeaufschlagung erhält man dann eine zusätzlich reinigende Wirkung aufgrund der Elektronenstrahlbeaufschlagung der Gasblasen. Es ist auch möglich, ändere reaktive Medien wie Halogene oder Wasserstoff oder Ammoniak einzusetzen.

Durch das erfindungsgemäße Verfahren lassen sich somit organische Verbindungen hocheffektiv zu anorganischen Verbindungen abbauen, wobei letztere wiederum ausgewaschen werden können oder sich neutralisieren lassen (insbesondere in-situ).

Entstehende gasförmige Spaltprodukte wiederum lassen sich durch den aktivierten Sauerstoff binden bzw. umwandeln.

Es kann eine effiziente Reinigungswirkung bei einer Vielzahl von unterschiedlichen Schadstoffverbindungen und Schadstoffgemischen erzielt werden; insbesondere lassen sich organische Verbindungen abbauen und biologisch aktive Substanzen abbauen.

Vorteilhaft ist es, wenn die Gaseinbringvorrichtung so ausgebildet ist, dass beim Einblasen eine Verwirbelung der Flüssigkeit erfolgt. Dadurch lässt sich die Eindringtiefe des Elektronenstrahls bei der Elektronenbestrahlung erhöhen und damit die Abbaurate bezüglich der Schadstoffe erhöhen.

Insbesondere ist dabei der Elektronenquelle eine weitere Verwirbelungsvorrichtung für die Flüssigkeit vorgeschaltet, um so den Verwirbelungsgrad zu erhöhen. Wenn die Verwirbelungsvorrichtung für die Flüssigkeit der Gaseinbringvorrichtung vorgeschaltet ist, dann kann über die Gaseinbringvorrichtung neben der Erzeugung der Zweiphasenströmung noch ein zusätzlicher Verwirbelungseffekt erreicht werden, über den wiederum die Eindringtiefe des Elektronenstrahls erhöht werden kann.

Ganz besonders vorteilhaft ist es, wenn durch die Gaseinbringvorrichtung ein reaktives Medium wie Luft und/oder Sauerstoff einblasbar ist. In einer Beaufschlagungszone der Elektronenquelle lassen sich dann nicht nur Schadstoffmoleküle direkt abbauen, das heißt in niedermolekulare und insbesondere anorganische Bestandteile zerlegen, sondern durch Wechselwirkung der Elektronen mit dem reaktiven Medium, wie beispielsweise Sauerstoff entstehen hochreaktives Ozon und/oder Sauerstoffradikale. Diese wiederum können Schadstoffmoleküle direkt angreifen und so die Abbaurate erhöhen. Man erhält dann eine Mehrfachwirkung der Elektronenbestrahlung, nämlich direkten Abbau der Schadstoffmoleküle und indirekten über die Erzeugung von hochreaktivern Ozon und/oder hochreaktiven Sauerstoffradikalen. Darüber hinaus lassen sich durch Sauerstoff und insbesondere aktivierten Sauerstoff gasförmige Reaktionsprodukte der Schadstoffmolekülzerlegung binden bzw. umwandeln.

Um eine hohe Energieeffizienz zu erhalten ist es vorteilhaft, wenn die Elektronenquelle einen gepulsten Elektronenstrahl erzeugt. Dadurch erhält man eine energieeffiziente Reinigungswirkung.

Wenn der Volumendurchsatz an Flüssigkeit so eingestellt ist, dass auf einen Volumenbereich eine Beaufschlagung durch mindestens zwei Elektronenstrahlpulse auf eine Lauflänge im Beaufschlagungsbereich der Elektronenquelle erfolgt, dann ist sichergestellt, dass ein genügend großer Wechselwirkungsquerschnitt (Streuquerschnitt) zwischen dem Elektronenstrahl und der Flüssigkeit beim Durchlaufen des Beaufschlagungsbereichs vorliegt, um so eine effiziente Reinigungswirkung zu erzielen.

Es kann auch vorgesehen sein, dass der Volumendurchsatz an Flüssigkeit und/oder eine Verwirbelung der Flüssigkeit so eingestellt ist, dass in einem Beaufschlagungsbereich der Elektronenquelle eine vollständige Umschichtung einer laminaren Strömung der Flüssigkeit oder eine im wesentlichen vollständige Durchmischung erfolgt. Dadurch wiederum wird sichergestellt, dass bezüglich des Gesamtvolumens der durch den Beaufschlagungsbereich

Die nachfolgende Beschreibung einer bevorzugten Ausführungsform dient im Zusammenhang mit der Zeichnung der näheren Erläuterung der Erfindung. Es zeigen:
- Figur 1: eine schematische Darstellung einer erfindungsgemäßen Vorrich- tung zur Reinigung einer schadstoffbelasteten Flüssigkeit, und
- Figur 2: eine schematische Darstellung verschiedener Mechanismen des Angriffs an Schadstoffmolekülen.

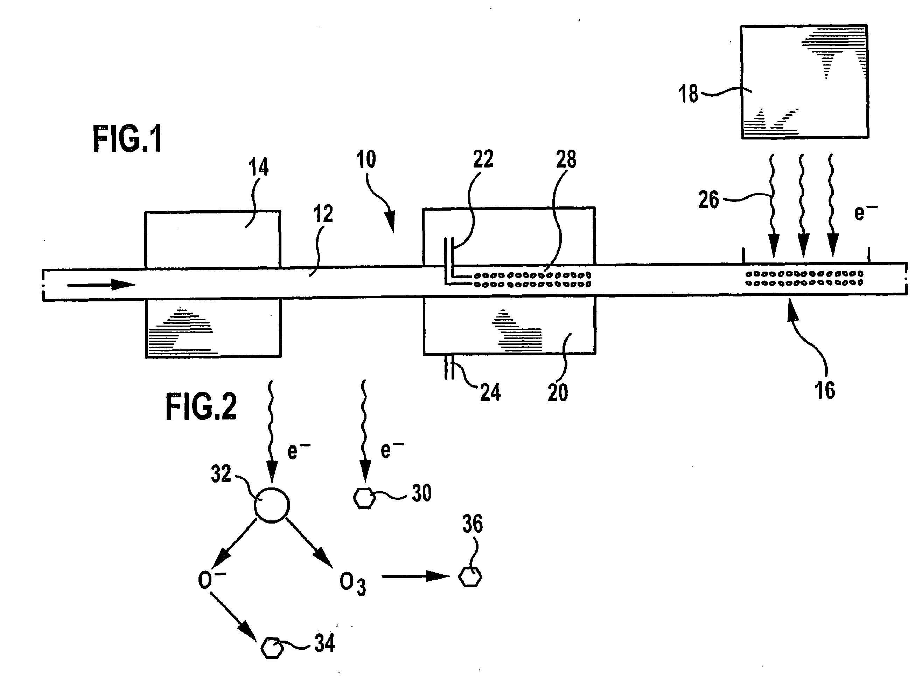

Ein Ausführungsbeispiel einer Vorrichtung, welches in Figur 1 als Ganzes mit 10 bezeichnet ist, umfasst eine Führung 12 in Form einer oder mehrerer Leitungen für die zu reinigende Flüssigkeit, bei der es sich beispielsweise um belastetes Abwasser wie Sickwasser aus einer Sondermülldeponie oder Hausmülldeponie enthalten kann. Die Flüssigkeit enthält Schadstoffe wie Fluor-Chlor-Kohlenwasserstoffe und weitere organische und metall-organische Verbindungen. Alternativ oder zusätzlich können auch biologisch aktive Substanzen enthalten sein; die Flüssigkeit ist dann zu sterilisieren.

Die Flüssigkeit ist in der Führung 12 laminar geführt und insbesondere in Form eines Flüssigkeitfilms geführt. Es sind entsprechende Vorrichtungen zur Erzeugung einer laminaren Strömung bzw. eines Flüssigkeitsfilms vorgesehen, welche in der Zeichnung nicht gezeigt sind. Filmdicken liegen im Millimeterbereich.

In der Führung 12 ist eine Verwirbelungsvorrichtung 14 angeordnet, welche dazu dient, den Flüssigkeitsfilm in der Führung 12 vor Eintritt in einen Beaufschlagungsbereich 16 einer Elektronenquelle 18 zu verwirbeln.

In Strömungsrichtung der Verwirbelungsvorrichtung 14 nachgeschaltet ist eine Gaseinbringvorrichtung 20, über welche sich beispielsweise mittels eines Einblasrohrs 22 Gas in die verwirbelte Flüssigkeit einblasen lässt. Die Gaselnbringvorrichtung 20 ist an eine Zuführungsleitung 24 gekoppelt, über welche das einzublasende Gas zugeführt wird.

Insbesondere wird reiner Sauerstoff oder Luft über die Gaseinbringvorrichtung 20 in den verwirbelten Flüssigkeitsfilm eingeblasen.

Der Gaseinbringvorrichtung 20 nachgeschaltet ist die Beaufschlagungszone 16 der Elektronenquelle 18. Die Elektronenquelle 18 erzeugt einen gepulsten Elektronenstrahl 26, welcher auf den Flüssigkeitsfilm in dem Beaufschlagungsbereich 16 wirkt.

Das erfindungsgemäße Verfahren funktioniert wie folgt:
Der Flüssigkeitsfilm wird in der Führung 12 dem Beaufschlagungsbereich 16 der Elektronenquelle 18 zugeführt. Durch die Verwirbelungsvorrichtung 14, welche beispielsweise in der Führung angeordnete Strömungswender umfasst, sorgt für eine Verwirbelung der Flüssigkeit.

Anschließend wird über die Gaseinbringvorrichtung 20 Gas eingeblasen, so daß in der Flüssigkeit Gasblasen 28 vorliegen. Dadurch wird zum einen die schadstoffbelastete Flüssigkeit zusätzlich verwirbelt und zum anderen wird eine Zweiphasenströmung erzeugt, das heißt die Strömung einer Flüssigkeit und eines Gases.

In dem Beaufschlagungsbereich 16 wird diese Zweiphasenströmung mit einem gepulsten Elektronenstrahl beaufschlagt. Gepulste Elektronenstrahlen haben eine Aufspaltungswirkung bezüglich Schadstoffmolekülen. Dies ist in Figur 2 schematisch angedeutet für ein Schadstoffmolekül 30: Durch Elektronenbeschuß kann das Schadstoffmolekül angeregt und anschließend aufgespaltet werden, so daß ein Schadstoffabbau erfolgt.

Als Gas wird vorteilhafterweise ein reaktives Medium eingeblasen, welches zum Schadstoffabbau beiträgt.

Insbesondere wenn Luft oder Sauerstoff über die Gaseinbringvorrichtung 20 sich in der Zweiphasenströmung befindet, dann wirken auch noch weitere Effekte: Tritt ein Elektron mit einer Gasblase 32 in Wechselwirkung, so kann dabei Ozon (O₃) und/oder es können Sauerstoffradikale (O⁻) entstehen. Sowohl Ozon als auch Sauerstoffradikale sind sehr reaktiv und können, wenn diese in Wechselwirkung mit Schadstoffmolekülen 34, 36 treten, zu deren Zerlegung führen.

Die Reinigungswirkung des Elektronenstrahls 26 an dem Flüssigkeitsfilm in dem Beaufschlagungsbereich 16 wird durch vorherige Verwirbelung dieses Flüssigkeitsfilms erhöht. Durch das Einblasen von Gas über die Gaseinbringvorrichtung 20 und Erzeugung einer Zweiphasenströmung wird dieser Reinigungsmechanismus noch weiter erhöht.

Durch das Einblasen von Luft und/oder Sauerstoff vor dem Beaufschlagungsbereich 16 durch die Gaseinbringvorrichtung 20 werden reaktives Ozon und/oder reaktive Sauerstoffradikale erzeugt, wodurch eine zusätzliche reinigende Wirkung entsteht, so daß insgesamt der Reinigungswirkungsgrad erhöht wird.

Es lassen sich dann organische Verbindungen 30, 34, 36 zu anorganischen Verbindungen abbauen, wobei diese anorganische Verbindungen sich dann neutralisieren lassen, insbesondere in-situ, oder sich auswaschen lassen.

Spaltprodukte der Schadstoffmoleküle 30, 34, 36, die gasförmig sind, lassen sich durch den aktivierten Sauerstoff binden bzw. umwandeln.

Aufgrund der verbesserten Verwirbelung läßt sich auch die Eindringtiefe des Elektronenstrahls 26 in dem Flüssigkeitsfilm erhöhen, bzw. es wird die Abbaurate erhöht.

Es ist vorgesehen, daß der Volumendurchsatz an Flüssigkeit durch die Führung 12 so eingestellt ist, daß während des Durchlaufens des Beaufschlagungsbereichs 16, das heißt während einer Lauflänge eines Flüssigkeitsvolumens durch diesen Beaufschlagungsbereich 16, mindestens zwei Elektronenstrahlpulse ausgesandt werden. Die Laufzeit eines Flüssigkeitsvolumens durch den Beaufschlagungsbereich 16 sollte deshalb mindestens zwei Periodendauern der Pulsung betragen.

Vorzugsweise ist der Volumenstrom an Gas und insbesondere Luft oder Sauerstoff, welcher durch die Gaseinbringvorrichtung in den Flüssigkeitsfilm eingeblasen wird, mindestens 10 % des Volumenstroms der Flüssigkeit in der Führung, um so eine effektive Verwirbelung und einen genügend großen Anteil von Gasblasen 32 der Flüssigkeit zu erhalten, um so wiederum neben der Ausbildung der Zweiphasenströmung und der effektiven Verwirbelung auch eine hohe Ausbeute für die lokale Erzeugung von Ozon und/oder Sauerstoffradikalen bei Elektronenbeaufschlagung im Beaufschlagungsbereich 16 zu erhalten.

## Patentansprüche

1. Verfahren zur Reinigung einer schadstoffbelasteten Flüssigkeit, bei dem die Flüssigkeit mit Elektronen bestrahlt wird und vor der Elektronenbestrahlung Gas in die Flüssigkeit eingeblasen wird, **dadurch gekennzeichnet, dass** die Flüssigkeit als Film einer Filmdicke im Millimeterbereich geführt wird, dass die Flüssigkeit vor der Gaseinblasung verwirbelt wird, dass das Gas zur Ausbildung einer Zweiphasenströmung eingeblasen wird und dass die Zweiphasenströmung mit den Elektronen bestrahlt wird.

2. Verfahren nach Anspruch 1, **dadurch gekennzeichnet, dass** Luft und/oder Sauerstoff eingeblasen wird.

3. Verfahren nach einem der Ansprüche 1 oder 2, **dadurch gekennzeichnet, dass** ein Gasvolumenstrom eingeblasen wird, der mindestens 10 % des Flüssigkeitsvolumenstroms beträgt.

4. Verfahren nach einem der Ansprüche 1 bis 3, **dadurch gekennzeichnet, dass** die Elektronenbeaufschlagung gepulst wird.

5. Verfahren nach Anspruch 4, **dadurch gekennzeichnet, dass** ein Flüssigkeitsvolumen während seiner Laufzeit durch einen Beaufschlagungsbereich mindestens zwei Elektronenstrahlpulse erfährt.

## Claims

1. Process for cleaning a contaminated liquid, in which the liquid is irradiated with electrons and prior to the electron irradiation gas is injected into the liquid, **characterized in that** the liquid is conveyed as a film of a film thickness in the millimetre range, **in that** the liquid is swirled, prior to the gas injection, **in that** the gas is injected to form a two-phase flow and **in that** the two-phase flow is irradiated with the electrons.

2. Process according to claim 1, **characterized in that** air and/or oxygen is injected.

3. Process according to one of claims 1 or 2, **characterized in that** there is injected a volumetric flow of gas that is at least 10% of the volumetric flow of liquid.

4. Process according to one of claims 1 to 3, **characterized in that** the impingement of electrons is pulsed.

5. Process according to claim 4, **characterized in that** a liquid volume during its transit time through an impingement region undergoes at least two electron beam pulses.

## Revendications

1. Procédé pour purifier un liquide chargé de substances nocives, dans lequel le liquide est irradié avec des électrons et, avant l'irradiation avec des électrons, un gaz est insufflé dans le liquide, **caractérisé en ce que** le liquide est guidé sous forme d'un film d'une épaisseur de film dans le domaine des millimètres, **en ce que** le liquide est soumis à un tourbillonnement avant l'insufflation de gaz, **en ce que** le gaz est insufflé pour la formation d'un écoulement à deux phases et **en ce que** l'écoulement à deux phases est irradié avec les électrons.

2. Procédé selon la revendication 1 **caractérisé en ce que** de l'air et/ou de l'oxygène est insufflé.

3. Procédé selon l'une des revendications 1 ou 2 **caractérisé en ce qu'**un courant volumique de gaz qui représente au moins 10 % du courant volumique de liquide est insufflé.

4. Procédé selon l'une des revendications 1 à 3 **caractérisé en ce que** l'application d'électrons est pulsée.

5. Procédé selon la revendication 4 **caractérisé en ce qu'**un volume de liquide subit au moins deux impulsions de faisceau d'électrons pendant sa durée de parcours dans un domaine d'application.
